# EUROPEAN PATENT SPECIFICATION

(11) **EP 3 658 607 B1**
(45) Date of publication and mention of the grant of the patent: **20.12.2023**
(21) Application number: 18755140.3
(22) Date of filing: 24.07.2018
(51) Int. Cl.: C08G 65/00, C08G 81/02, C08G 65/325, C08G 65/331, C08G 65/332, C08G 65/334, C08G 65/34

(54) **DIBLOCK COPOLYMER, A MANUFACTURING METHOD AND SUITED APPLICATIONS**
DIBLOCKCOPOLYMER, HERSTELLUNGSVERFAHREN UND GEEIGNETE ANWENDUNGEN
COPOLYMÈRE DIBLOC, UN PROCÉDÉ DE FABRICATION ET DES APPLICATIONS APPROPRIÉES

(30) Priority: 24.07.2017 EP 17182789
(43) Date of publication of application: 03.06.2020
(73) Proprietor: Freie Universität Berlin, 14195 Berlin (DE); President And Fellows Of Harvard College, Cambridge, Massachusetts 02138 (US)
(72) Inventor: HAAG, Rainer, 12209 Berlin (DE); CHOWDHURY, Mohammad Suman, 10555 Berlin (DE); WEITZ, David, Bolton Massachusetts 01740 (US)
(74) Representative: Maikowski & Ninnemann Patentanwälte Partnerschaft mbB
(86) International application number: PCT/EP2018/070036
(87) International publication number: WO 2019/020626

(56) References cited:
- WO-A1-2015/164781
- US-A1- 2010 240 560
- US-A1- 2012 190 603
- WAGNER, O.; THIELE, J.; WEINHART, M.; MAZUTIS, L.; WEITZ, D. A.; HUCK, W. T.; HAAG, R.: "Biocompatible fluorinated polyglycerols for droplet microfluidics as an alternative to PEG-based copolymer surfactants", LAB ON A CHIP, vol. 16, no. 1, 2016, pages 65-69, XP002775855, cited in the application
- DATABASE CAPLUS [Online] CHEMICAL ABSTRACTS SERVICE, COLUMBUS, OHIO, US; XI: "Fluoro compound block polyether-diisocyanate reaction products as demulsifiers in petroleum recovery and for petroleum", XP002775856, Database accession no. 2006:96951
- DATABASE WPI Week 201206 Thomson Scientific, London, GB; AN 2011-P11724 XP002775857, & CN 102 210 995 A (UNIV ZHEJIANG TECHNOLOGY) 12 October 2011 (2011-10-12)

## Description

### FIELD OF INVENTION

The present invention relates to a diblock copolymer according to the preamble of claim 1, to a method for manufacturing such a diblock copolymer according to the preamble of claim 9, to a droplet made of such a diblock copolymer according to the preamble of claim 12 as well as to uses of such a droplet according to the preambles of claims 14 and 15.

### BACKGROUND

US 9,012,390 B2 and US 9,498,761 B2 describe surfactants, in particular fluorosurfactants, for stabilizing aqueous or hydrocarbon droplets in a fluorophilic continuous phase. These surfactants comprise a block copolymer including a perfluorinated polyether (PFPE) block coupled to a polyethylene glycol (PEG) block via an amide bond.

From the limited library of fluorinated surfactants the perfluoropolyether-polyethylene glycol-perfluoropolyether (PFPE-PEG-PFPE) triblock copolymer is the benchmark for the formulation and stability of water-in-oil (W/O) and water-in-oil-in-water (W/O/W) emulsions, particularly for biological applications due to its biocompatibility. However, it has several limitations, for example, droplet coalescence at higher temperature, cross-contamination of microreactors (droplets), instability of droplets after a certain threshold of volume fraction, false optical read out in intensity derived fluorescence assay as droplets shrink over a short amount of time, etc. Most importantly, the molar mass dependency of both PEG and PFPE blocks for surfactant synthesis frequently leads to undesirable batch to batch variation.

To overcome some limitations of the PEG-based block copolymers, a triblock copolymer having a central linear polyglycerol block and two flanking PFPE blocks has been developed and described by Wagner et al. (Wagner, O., Thiele, J., Weinhart, M., Mazutis, L., Weitz, D. A., Huck, W. T., & Haag, R. (2016). Biocompatible fluorinated polyglycerols for droplet microfluidics as an alternative to PEG-based copolymer surfactants. Lab on a Chip, 16(1), 65-69).

Further examinations revealed that these triblock copolymers have an insufficient thermal stability.

WO 2015/164781 A1 discloses an emulsion for delivery of a therapeutic agent, wherein the emulsion comprises semi-fluorinated block copolymers, wherein each of these block copolymers comprises a hydrophilic block, a hydrophobic block and fluorophilic block.

US 2010/0240560 A1 discloses an ether composition and a lubricant which bond to substrates with a high bonding ratio and form a coating having a low friction coefficient surface.

US 2012/0190603 A1 discloses an ether compound which is capable of maintaining high adhesion to a base material even in the presence of moisture. This ether compound can be used as lubricant.

Chemical abstracts accession number 2006:969651 discloses a fluoro compound block polyether-diisocyanate reaction products as demulsifiers in petroleum recovery and for petroleum. Thus, these compounds are particularly useful for destabilizing emulsions.

Chemical abstracts accession number 2011-P11724 relates to a polyglycerol perfluoro alkenyl ether mixture that can be used as surfactant. Dimethylamine or trimethylamine is used as catalyst for a synthesis reaction of these compounds.

### SUMMARY

It is an object of the present invention to provide novel compounds that can be used for forming droplets in water-in-oil (W/O) and water-in-oil-in-water (W/O/W) emulsions, wherein these droplets have at least a high thermal stability.

This object is achieved by a diblock copolymer having the features of claim 1. Such a diblock copolymer consists of a first block, a second block, and a linker. Thereby, the second block is covalently bound to the first block by the linker. The first block is a glycerol block. It comprises 1 to 10 glycerol subunits that are optionally substituted. If the number of glycerol subunits increases beyond the before-mentioned upper limit, the properties of the diblock copolymer changes so that droplets formed by such diblock copolymers do not have stability of the droplets formed by diblock copolymers complying with the definitions of claim 1.

The second block is a superhydrophobic block comprising a perfluoroether residue having at least 20 carbon atoms. Thereby, at least 40 % of all possible substitution sites (e.g., a methylene bridge has two substitution sites and a methyl end group of a hydrocarbon chain has three substitution sites) are substituted by fluorine. Due to such substitution by fluorine, the hydrocarbon chain of the superhydrophobic block does not only exhibit hydrophobic properties, but rather superhydrophobic properties.

The specific combination of a glycerol block having only a small number of glycerol units and superhydrophobic hydrocarbon block with a minimum of 20 carbon atoms results in a diblock copolymer having superior properties as compared to compounds known from prior art. Specifically, droplets made of these diblock copolymers show a high thermal stability so that droplets made of these diblock copolymers can be used as mini reactors for high-temperature applications, such as PCR. Likewise, the droplets can well be used for screening of small molecules, such as drugs.

The novel diblock copolymers can also be denoted as glycerol-based fluorosurfactants. They can be applied to stabilize W/O and W/O/W emulsions. Non-limiting examples of applications of these emulsions include single cell DNA analysis by polymerase chain reaction (PCR), evolution of enzyme function, cell cytotoxicity test in microdroplets, and high throughput screening of low molecular weight molecules. Surprisingly, droplets formed by the claimed diblock copolymer showed almost no cross-contamination by reagents contained within the droplets. Expressed in other words, the droplets did on the one hand not release (to a significant extent) reagents contained in their interior and did on the other hand not take up (to a significant extent) reagents from their surroundings. This extremely low cross-contamination capability makes droplets formed by the claimed diblock copolymer particularly appropriate for encapsulating small molecules used in the water phase.

Essentially, the fluorophilic compounds for the tail of this surfactant (building the superhydrophobic block) can be any available perfluoropolyether that contains a carboxylic group at its terminus. Non-limiting examples are fluorinated alkyl chains of any size and/or length with linear, hyperbranched, branched, saturated, unsaturated, dendritic, cyclic, homobifunctional, or heterobifuctional architectures. Appropriate PFPE polymers for building the superhydrophobic block are manufactured by DuPont under the trade names Krytox^{®} 157 FSH, Krytox^{®} 157 FSM, and Krytox^{®} 157 FSL.

On the other hand, the head group (building the glycerol block) in the novel amphiphilic surfactants is an oligo-glycerol derivative, wherein the number of glycerol units varies from 1 to 10. The head group of the surfactant make the surfactant water soluble, hygroscopic and biocompatible. Essentially, the hydrophilic oligo-glycerol head group can be either one of its enantiomers (*R,R* and *S,S*)*,* a mixture of enantiomers, meso-form or a mixture of individual stereoisomers. The hydroxyl groups in the head groups of the surfactant can be pre-modified and/or post-modified to render it soluble in organic solvent. Any anionic, cationic, non-ionic, and zwitterionic compound can be coupled to the head group during pre-/post- modification.

The head group (glycerol block) is coupled to the fluorophilic tail (superhydrophobic block) *via* a linker forming any covalent bond. More specifically, the coupling chemistry can include any kind of click chemistry, amide bond formation, esterfication, Friedel-Crafts alkylation, but is not limited to these synthesis reactions.

As mentioned, the molar mass dependence of the benchmark surfactant renders the formulation uncontrolled from batch to batch. If no stoichiometric reaction is achieved, a mixture of di-block and tri-block co-polymers, as well as homo-polymers of both hydrophilic and fluorophilic groups results. On the contrary, even though there is a distribution of molar mass of commercially available PFPE, the flexibility on stoichiometric reaction conditions makes the synthesis of diblock copolymers as described herein not only more controlled but also superior over the synthesis of PFPE-PEG-PFPE surfactants. Thereby, an aqueous or organic medium can be used as dispersed phase to solubilize the glycerol block. The fluorinated oils HFE 7500 (hydrofluoroether 7500, molecular weight 414 g/mol) and FC40 can be very well used to prepare the continuous phase to solubilize the superhydrophobic block of the diblock copolymer.

The glycerol subunits of the glycerol block can be substituted by any residues that are usually used for substituting polyglycerol, such as thiol, methyl, ethyl, amine, methoxy, ethoxy, methylethylether, phosphate, sulfate, phosphate choline, and choline phosphate residues. In an embodiment, however, the glycerol subunits of the glycerol block are non-substituted, i.e., they carry only hydrogen and hydroxyl residues. A diblock copolymer with such non-substituted glycerol subunits in the glycerol block exhibits higher biocompatibility as compared to a diblock copolymer with substituted glycerol subunits.

While a diblock copolymer comprising only a single glycerol subunit (monoglycerol) or two glycerol subunits (diglycerol) is principally possible, the positive properties of the diblock copolymer are particularly well achieved if an oligoglycerol comprising 3 to 10 glycerol subunits makes up the first block. Therefore, in an embodiment, the glycerol block comprises 3 to 10 optionally substituted glycerol subunits, such as 3, 4, 5, 6, 7, 8, 9 or 10 glycerol subunits. Thereby, 3 or 7 glycerol subunits are particularly appropriate to build up the glycerol block. Thus, the oligoglycerol can, in an embodiment, comprise 3 to 7 glycerol subunits.

In an embodiment, the oligoglycerol is a branched oligoglycerol. It turned out that a diblock copolymer comprising a branched oligoglycerol can particularly well stabilize droplets made of such a diblock copolymer. Thereby, the term "branched" refers to an arrangement in which the individual glycerol subunits are not linearly connected to each other. Furthermore, the superhydrophobic block is connected to the glycerol block by a carbon atom of the glycerol block that is not a terminal carbon atom of any of its glycerol subunits.

In an embodiment, the glycerol block corresponds to general formulae (I), (II), (III), (IV), (V), (VI), (VII), or (VIII):

| | |
|---|---|
| 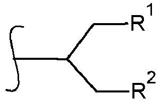 | (I) |
| 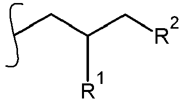 | (II) |
| 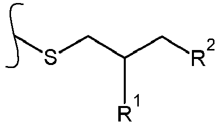 | (III) |
| 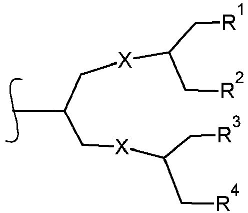 | (IV) |
| 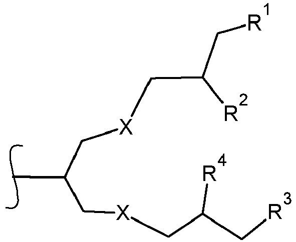 | (V) |
| 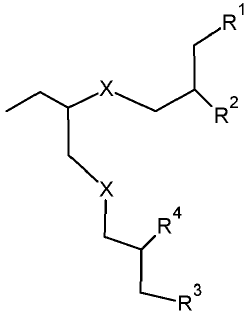 | (VI) |
| 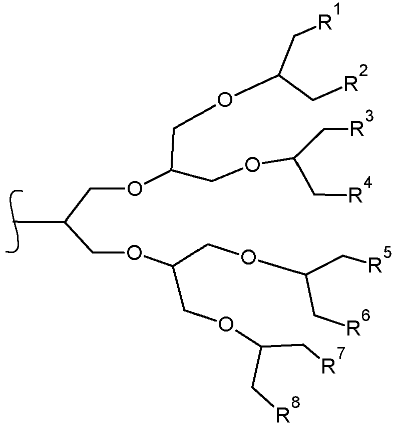 | (VII) |
| 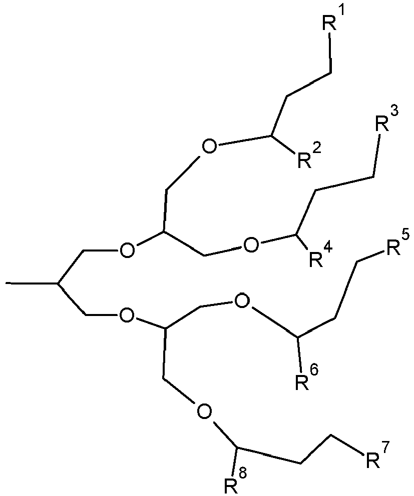 | (VIII) |

Thereby, R¹, R², R³, R⁴, R⁵, R⁶, R⁷, R⁸ denote independently from each other, -SH, -CH₃, -CH₂CH₃, -NH₂, -OH, -OCH₃, -OCH₂CH₃, -OCH₂OCH₂CH₃, -NH₃⁺, -OPO₃⁻(CH₂)₂N⁺(CH₃)₃, -(CH₃)₂N⁺(CH₂)₂OPO₂⁻(OCH₃), -(CH₃)₂N⁺(CH₂)₂OPO₂⁻(OH), or -OSO₃⁻.

Furthermore, X means O or S. O is particularly appropriate.

Formulae (I) to (III) describe a G0 glycerol (derivative). Formulae (IV) to (VI) describe a branched G1 oligoglycerol (derivative) comprising three glycerol residues. Formulae (VII) and (VIII) describe a branched G2 oligoglycerol (derivative) comprising seven glycerol residues.

In an embodiment, the glycerol block corresponds to the following structure (IIa) falling under general formula (II):

| | |
|---|---|
| 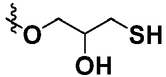 | (IIa) |

In an embodiment, the glycerol block corresponds to the following structure (IIb) falling under general formula (II):

| | |
|---|---|
| 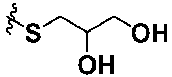 | (IIb) |

In an embodiment, the superhydrophobic block comprises at least 22, 24, 26, 28, 30, 35, 40, 45, or 50 carbon atoms. In an embodiment, it comprises 20 to 50 carbon atoms, in particular 22 to 45, in particular 24 to 40, in particular 26 to 35, in particular 28 to 30 carbon atoms. These carbon atoms can be arranged in a single hydrocarbon chain. However, such an arrangement is not mandatory. Rather, it is possible that the carbon atoms of the superhydrophobic block are arranged in more than one hydrocarbon chain, such as two or three hydrocarbon chains. Thereby, the number of chains depends on the chemical nature of the linker used to couple the second block to the first block.

In an embodiment, the superhydrophobic block comprises two carbon-containing chains (such as hydrocarbon chains) that are bound to the same linker. Thereby, these carbon-containing chains are not covalently cross-linked to each other. Rather, the only covalent connection between the first carbon-containing chain and the second carbon-containing same is via the linker to which both chains are covalently bound.

In an embodiment, at least 45%, in particular at least 50%, in particular at least 55%, in particular at least 60%, in particular at least 65%, in particular at least 70%, in particular at least 75%, in particular at least 80%, in particular at least 85%, in particular at least 90%, in particular at least 95% and very particular all substitution sites of the carbon atoms of the carbon-containing chain of the superhydrophobic block are substituted by fluorine. In an embodiment, 40 % to 100 %, in particular 45 % to 95 %, in particular 50 % to 90 %, in particular 55 % to 85 %, in particular 60 % to 80 %, in particular 65 % to 75 % of all substitution sites of the carbon atoms of the carbon-containing chain of the superhydrophobic block are substituted by fluorine.

If the superhydrophobic block comprises more than one carbon-containing chain, all carbon-containing chains have, in an embodiment, the same number of carbon atoms and, in another embodiment, the same overall chemical structure.

In an embodiment, the superhydrophobic block comprises a perfluoroether (PFPE) residue, in particular a PFPE residue as described in US 9,012,390 B2, as long as it complies with constraints of a minimum number of carbon atoms as defined above.

In an embodiment, the superhydrophobic block comprises a residue according to general formulae (IX) or (X):

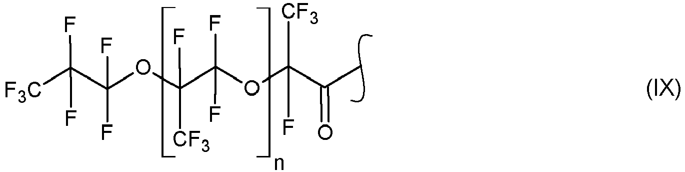

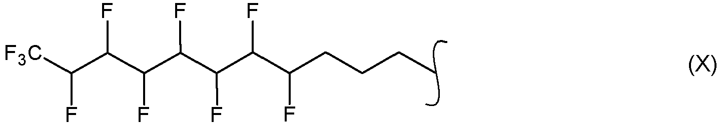

Thereby, n is an integer between 10 and 50.

In an embodiment, n is 10 to 20, in particular 10 to 18, in particular 10 to 16, in particular 10 to 14. In another embodiment, n is 20 to 30, in particular 22 to 28, in particular 24 to 26. In another embodiment, n is 30 to 50, in particular 32 to 48, in particular 34 to 46, in particular 36 to 44, in particular 38 to 42.

In an embodiment, the linker is at least one chosen from the group consisting of amines, amides, oxygen (i.e. an ether bridge), and an alkyl residue having 1 to 20 carbon atoms (or 2 to 20 carbon atoms), wherein a hydrocarbon chain of the alkyl residue can be interrupted by one or more oxygen, nitrogen and/or sulfur atoms and/or can be substituted by a group comprising one or more oxygen, nitrogen and/or sulfur atoms.

In an embodiment, the linker is not a methylene group. In this embodiment, It be any other chemical group, e.g., a group as specified in the preceding paragraph except methylene.

In an aspect, the instant invention also relates to a method for manufacturing a diblock copolymer as defined above. This manufacturing method comprises a step of reacting an oligoglycerol amine comprising protected hydroxyl groups with a perfluorinated polyether acid chloride in an organic solvent, and deprotecting the hydroxyl groups of the obtained diblock copolymer. The organic solvent comprises, in an embodiment, a hydrofluoroether such as HFE-7100 or HFE-7500. The organic solvent comprises, in an embodiment, dichloromethane. A mixture of a hydrofluoroether and dichloromethane is particularly appropriate as organic solvent. The deprotection can be carried out, in an embodiment, under acidic conditions. An acetal protecting group is an appropriate protecting group. Appropriate reaction temperatures lie in a range between 40 and 55 °C, in particular between 45 and 50 °C.

In an embodiment, the oligoglycerol amine comprising protected hydroxyl groups is manufactured by mesylating an oligoglycerol comprising protected hydroxyl groups and at least one unprotected hydroxyl group to introduce a mesyl group into the oligoglycerol. An appropriate mesylation compound is methane sulfonyl chloride. Appropriate reaction temperatures lie in a range between 0 °C and 30 °C, in particular between 10 and 25 °C. The mesyl group is then replaced by an azide group. This is done, in an embodiment, by reacting the mesylated oligoglycerol with an azide compound such as sodium azide. Dichloromethane is an appropriate solvent for such an azidation. Appropriate reaction temperatures lie in a range between 50 and 70 °C, in particular between 55 and 65 °C. Finally, the azide group is hydrogenated to obtain the oligoglycerol amine comprising protected hydroxyl groups. Hydrogenation is carried out, in an embodiment, with the help of a catalyst such as a palladium catalyst or a palladium/carbon catalyst. Methanol is an appropriate solvent for such a hydrogenation. Appropriate reaction temperatures lie in a range between 0 °C and 30 °C, in particular between 10 and 25 °C.

In an embodiment, the perfluorinated polyether acid chloride is manufactured by reacting a perfluorinated polyether with oxalyl chloride in an organic solvent. The organic solvent comprises, in an embodiment, a hydrofluoroether such as HFE-7100 or HFE-7500. The organic solvent comprises, in an embodiment, dichloromethane. A mixture of a hydrofluoroether and dichloromethane is particularly appropriate as organic solvent. This organic solvent can be identical or different to the organic solvent used for reacting the oligoglycerol amine comprising protected hydroxyl groups with the perfluorinated polyether acid chloride. Appropriate reaction temperatures lie in a range between 0 °C and 30 °C, in particular between 10 and 25 °C.

In another aspect, the present invention relates to a droplet made of a plurality of diblock copolymers according to the preceding explanations. Such a droplet typically comprises a single layer of diblock copolymers. If this droplet is formed in an oily medium, such as a perfluorinated oil, e.g., a short-chained perfluorinated oil, the second block (superhydrophobic block) is arranged to an outside of the droplet, wherein the first block (glycerol block) is arranged to an inside of the droplet. Then, a hydrophilic phase, such as an aqueous phase, can be incorporated within the interior of the droplet. To give an example, a buffer system in an aqueous phase such as water can be present in the inside of a corresponding droplet. A culture medium is also a suited aqueous phase to be incorporated into a corresponding droplet.

In an embodiment, the droplet does not comprise only diblock copolymers of the same structure, but structurally different diblock copolymers. To give an example, the droplets may comprise a first diblock copolymer comprising a low molecular weight PFPE and a second diblock copolymer comprising a medium molecular weight PFPE. Similarly, the droplet might comprise a second diblock copolymer comprising a medium molecular weight PFPE and a third diblock copolymer comprising a high molecular weight PFPE. Likewise, if a mixture of a first diblock copolymer comprising a low molecular weight PFPE and a third diblock copolymer comprising a high molecular weight PFPE is also possible. Furthermore, a droplet may be composed of more than two structurally different diblock copolymers. To give an example, such droplets might comprise a first diblock copolymer, a second diblock copolymer and a third diblock copolymer according to the preceding definitions.

The term "low molecular weight" with respect to the PFPE residue of the diblock copolymer relates, in an embodiment, to a PFPE residue (or superhydrophobic block) having a molecular weight of 1500 to 3000 g/mol. If the superhydrophobic block corresponds to general formula (IV), such molecular weight is achieved if n is 10 to 16 (or an integer between these thresholds).

The term "medium molecular weight" with respect to the PFPE residue of the diblock copolymer relates, in an embodiment, to a PFPE residue (or superhydrophobic block) having a molecular weight of 3500 to 5000 g/mol. If the superhydrophobic block corresponds to general formula (IV), such molecular weight is achieved if n is 22 to 28 (or an integer between these thresholds).

The term "high molecular weight" with respect to the PFPE residue of the diblock copolymer relates, in an embodiment, to a PFPE residue (or superhydrophobic block) having a molecular weight of 5500 to 8000 g/mol. If the superhydrophobic block corresponds to general formula (IV), such molecular weight is achieved if n is 34 to 46 (or an integer between these thresholds).

In an aspect, the invention relates to an emulsion of an aqueous dispersed phase, a continuous phase comprising a fluorinated oil, and a surfactant comprising a diblock copolymer according to the preceding explanations. Typically, the surfactant serves for droplet formation and/or stabilization of the dispersed phase in the continuous phase.

In an embodiment, the dispersed phase has an average diameter bigger than or equal to about 20 nm and less than or equal to about 200 microns.

In an embodiment, the dispersed phase has an average diameter of no more than about 200 microns.

In an embodiment, the dispersed phase has an average diameter bigger than or equal to about 20 nm.

In an embodiment, the dispersed phase comprises an aqueous solution comprising a biological molecule. In an embodiment, the biological molecule comprises a nucleic acid. In an embodiment, the biological molecule comprises an oligonucleotide.

In an embodiment, the dispersed phase comprises at least one of buffers, salts, nutrients, therapeutic agents, drugs, hormones, antibodies, analgesics, anticoagulants, antiinflammatory compounds, antimicrobial compositions, cytokines, growth factors, interferons, lipids, oligonucleotides polymers, polysaccharides, polypeptides, protease inhibitors, cells, nucleic acids, RNA, DNA, vasoconstrictors or vasodilators, vitamins, minerals, and stabilizers.

In an aspect, the present invention also relates to a method for performing a chemical and/or biological reaction in the dispersed phase according to the preceding explanations.

In an aspect, the present invention also relates to the use of the droplets according to the preceding explanations for performing a PCR in an interior of the droplets. As outlined above, the droplets formed by the instantly claimed diblock copolymers have a very high thermal stability. Therefore, they can well be used as mini reactors for performing a PCR in their interior. When heating up an emulsion of corresponding droplet to temperatures being typical for a PCR (up to ca. 98 °C), the droplets remain stable and do not disintegrate. Thus, all components being composed within the interior of the droplets remain in the interior and can be used for reactions such like the reactions to be typically performed during a PCR (i.e., denaturation, annealing and extension).

In an aspect, the instant invention also relates to a method for performing a PCR in an interior of a droplet according to the preceding explanations.

In an aspect, the instant invention also relates to the use of a droplet according to the preceding explanations for testing in vitro an effect of a substance on a cell incorporated in the droplet. Thereby, the substance can be, in an embodiment, a pharmaceutically active substance, such as a pharmaceutically active small molecule. It is also possible to incorporate more than one substance into the droplet. Thus, a combination of pharmaceutically active substances can be incorporated. For such a test of one or more substances in a single droplet, a cell like a tumor cell can be incorporated together with culture medium and the pharmaceutically active substance within an interior of the droplet formed by at least one type of diblock copolymer according to the preceding explanations. Afterwards, the cell viability within the droplet can be determined and compared with the cell viability of a cell incorporated in a droplet containing the same culture medium in its interior, but no pharmaceutically active substance. By such an experimental setup, which can be realized by a plurality of microfluidic systems arranged in parallel for producing droplets with incorporated cells and optionally a substance to be tested, a high number of pharmaceutically active substances can be tested with respect to their effect on the viability of cells, regulation of gene expression, alteration of genotype and phenotype of cells (or other properties).

In an aspect, the instant invention relates to a method for testing an effect of the substance on a cell incorporated in a droplet comprising a plurality of diblock copolymers according to the preceding explanations.

All embodiments described herein can be combined in any desired way. Furthermore, embodiments described with respect to the diblock copolymer can be transferred to any of its manufacturing method, the droplet made of a plurality of diblock copolymers and the uses of the droplet, and vice versa. Likewise, embodiments of any described subject matter can be transferred to any other subject matter described herein.

### BRIEF DESCRIPTION OF THE DRAWINGS

Further details of aspects of the present invention will be explained with respect to exemplary embodiments and accompanying Figures. In the Figures:
- Figure 1A: shows droplets made from a PFPE-PEG-PFPE triblock copolymer according to US 9,012,390 B2 after performing a PCR at 95 °C;
- Figure 1 8: shows droplets made from a PFPE-LPG(OMe)-PFPE triblock copolymer according to Wagner et al. after performing a PCR at 95 °C;
- Figure 1C: shows droplets made from an embodiment of a diblock copolymer as described herein after performing a PCR at 95 °C;
- Figure 2A: shows a size distribution of the droplets depicted in Figure 1A;
- Figure 2B: shows a size distribution of the droplets depicted in Figure 1B;
- Figure 2C: shows a size distribution of the droplets depicted in Figure 1C;
- Figure 3A: shows different panels depicting differently composed droplets with and without fluorescence dye directly after generating the droplets (0 hours) and after an incubation period of 72 hours;
- Figure 3B: shows droplets stabilized by LG0 with and without fluorescence dye after an incubation period of 72 hours;
- Figure 3C: shows droplets stabilized by MG0 with and without fluorescence dye after an incubation period of 72 hours;
- Figure 4: shows a plot on the cell viability of a leukemia cell line incorporated within droplets of different composition;
- Figure 5: shows a reaction scheme of an embodiment of a method for synthesizing a protected oligoglycerol amine;
- Figure 6: shows a reaction scheme of an embodiment of a method for chlorinating a perfluorinated polyether;
- Figure 7: shows a reaction scheme of an embodiment of a method for manufacturing a diblock copolymer starting from a protected oligoglycerol amine and a perfluorinated polyether chlorate;
- Figure 8A: shows FT-IR spectra of embodiments of diblock copolymers having three glycerol units;
- Figure 8B: shows FT-IR spectra of embodiments of diblock copolymers having one glycerol unit;
- Figure 9A: shows a reaction scheme for the synthesis of 1,3-bis((2,2-dimethyl-1,3-oxathiolan-5-yl)methoxy)propan-2-ol;
- Figure 9B: shows an 1H-NMR spectrum of the acetal-protected 1,3-bis((2,2-dimethyl-1,3-oxathiolan-5-yl)methoxy)propan-2-ol;
- Figure 10A: shows size distributions of droplets prepared of embodiments of a diblock copolymer before performing a PCR test;
- Figure 10B: shows microscopic pictures of the droplets, the size distributions of which are depicted in Figure 10A;
- Figure 10C: shows size distributions of droplets of the same diblock copolymers as in Figure 10A after performing a PCR test;
- Figure 10D: shows microscopic pictures of the droplets, the size distributions of which are depicted in Figure 10C;
- Figure 11A: shows microgels formed with the help of diblock copolymers serving as surfactants before surfactant removal (lower panels) and after surfactant removal (upper panels);
- Figure 11B: shows the size distribution of microgel particles when MG1 is used as surfactant;
- Figure 11C: shows the size distribution of microgel particles when HG1 is used as surfactant;
- Figure 11D: shows the size distribution of microgel particles when LG1 is used as surfactant;
- Figure 11E: shows precursor macromolecules that were used to prepare the microgel templates and a reaction scheme of the applied chemical reaction;
- Figure 12A: shows a fluorescence image of mixed drop populations of a cross-talking experiment;
- Figure 12B: shows an overlay of bright field and fluorescence of the mixed drop populations of Figure 12A;
- Figure 12C: shows a fluorescence image of the positive control of the cross-talking experiment; and
- Figure 12D: shows an overlay of bright field and fluorescence of the positive control of Figure 12C.

### DETAILED DESCRIPTION

The Figures will now be explained with respect to exemplary embodiments.

### Example 1: Temperature stability

The diblock copolymers described herein have the capability to stabilize emulsions even at high temperatures which can be essential for performing assays in droplets. For example, polymerase chain reaction (PCR) is the most widely used method for DNA amplification which is an indispensable constitute for drop-based biological assays. However, PCR requires as high as 98 °C incubation temperature in amplification cycles. At this high temperature, emulsions with PCR reagents stabilized by PFPE-PEG-PFPE triblock copolymers known from prior art fail to remain stable and coalesce.

Monodisperse PCR reagent droplets having a diameter of about 75 µm stabilized by 2% (w/w) surfactant in HFE 7500 solution were tested. The following surfactants were used for building up the droplets:
a) PFPE-PEG-PFPE triblock copolymer as described in US 9,012,390 B2 (Krytox-PEG-Krytox);
b) PFPE-LPG(OMe)-PFPE triblock copolymer as described in Wagner et al. (Krytox-LPG(OMe)-Krytox);
c) PFPE-PG diblock copolymer according to the following formula (XI) (G1-Krytox; LG1): 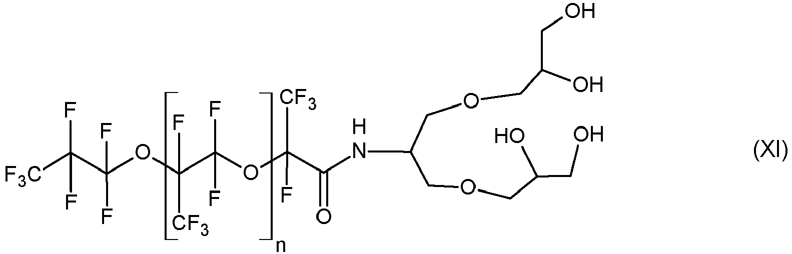
with n = 10 to 16

The used PCR kit was the Phusion kit from Thermo Fisher with detergent-free buffer. Lambda Phage genome fragmented and tagged by Nextera Kit was used as template.

After performing 35 PCR cycles with 98 °C denaturation temperature (whole cycling conditions: 98 °C for 30 s, then 35 cycles of: 98 °C for 7 s, 60 °C for 30 s, 72 °C for 20 s. After cycling, 72 °C for 10 min), about 25% of emulsions stabilized by PFPE-PEG-PFPE merged into a water layer while only 10% of emulsion stabilized by LG1 merged into water layer.

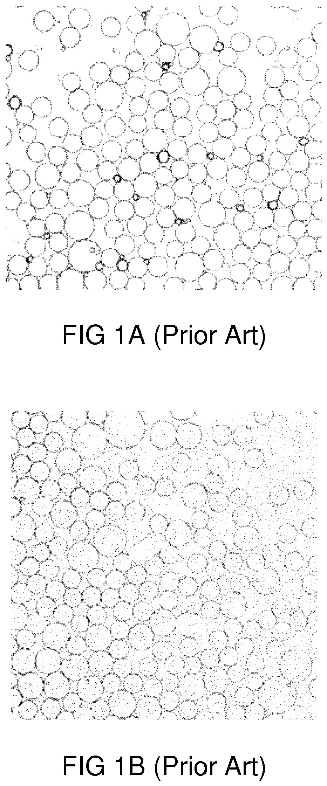

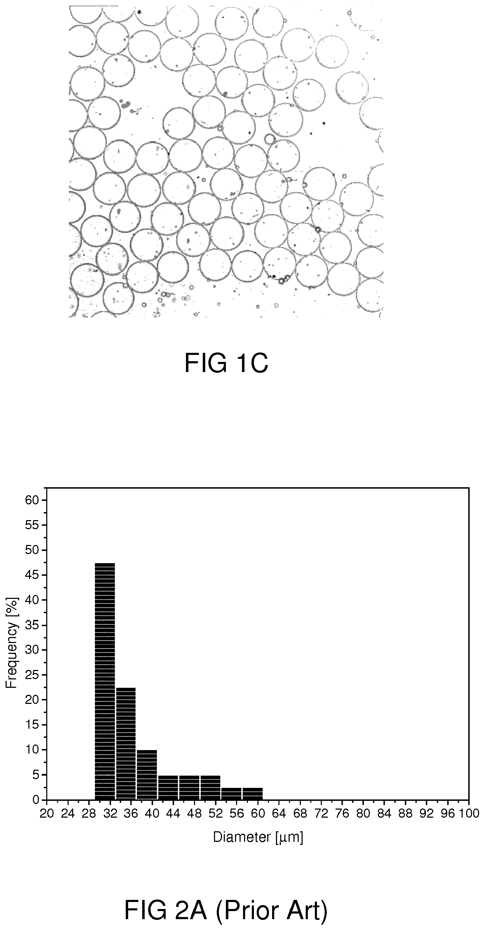

The droplets that survived the PCR cycles were examined under microscope. Droplets stabilized by PFPE-PEG-PFPE (Figure 1A) and PFPE-LPG(OMe)-PFPE (Figure 1B) were no longer monodisperse. This clearly indicates that they were destabilized due to the temperature treatment. Droplets stabilized by LG1 remained, however, monodisperse (Figure 1C).

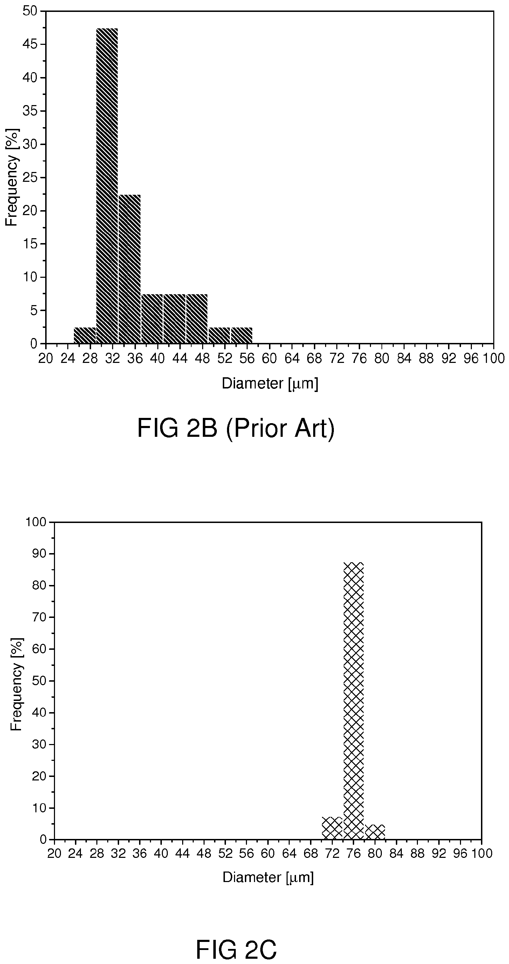

The size of the droplets stabilized by different surfactants was examined in more detail by measuring the sizes of the droplets depicted in Figures 1A to 1C and assigning a size distribution to the corresponding droplets. The results are shown in Figures 2A to 2C. PFPE-PEG-PFPE (Figure 2A) and PFPE-LPG(OMe)-PFPE (Figure 2B) show a broad size distribution in a diameter range of approximately 25 to 60 µm with a highest incidence in both cases at 29 to 33 µm. In contrast, the droplets stabilised by LG1 only show a very narrow size distribution in a diameter range of 70 to 82 µm with a highest incidence at 74 to 78 µm (Figure 2C).

### Example 2: Cross-talking among droplets

As a superb platform for precisely miniaturizing and compartmentalizing chemical assays, droplet-based microfluidics is of particular interest in a number of biotechnological applications. The technology essentially utilizes water-in-oil emulsions as droplet microreactors, typically at nanoliter or picoliter scale. The extent of application is, however, limited by the so-called cross-talking between droplets. Cross-talking denotes molecular transport between droplets, leading to an inaccuracy of the droplet-based assays. As driven by the thermodynamic disequilibrium of aqueous/oil interfaces, the cross-talking phenomenon is also affected by the type of surfactant used to stabilize the emulsion.

To test the cross-talking phenomenon, two kinds of aqueous droplets were made and incubated for 72 hours: smaller droplets incorporating fluorescent dye dissolved in phosphate-buffered saline (PBS) (positive droplets) and larger droplets incorporating only PBS without dye (negative droplets). These two types of droplets were prepared with different droplet-stabilizing surfactants using a surfactant concentration of 2% (w/w) in a HFE 7500 solution.

Images of the droplets were taken immediately after generating the droplets (0 hours) and after an incubation period of 72 hours using a confocal microscope.

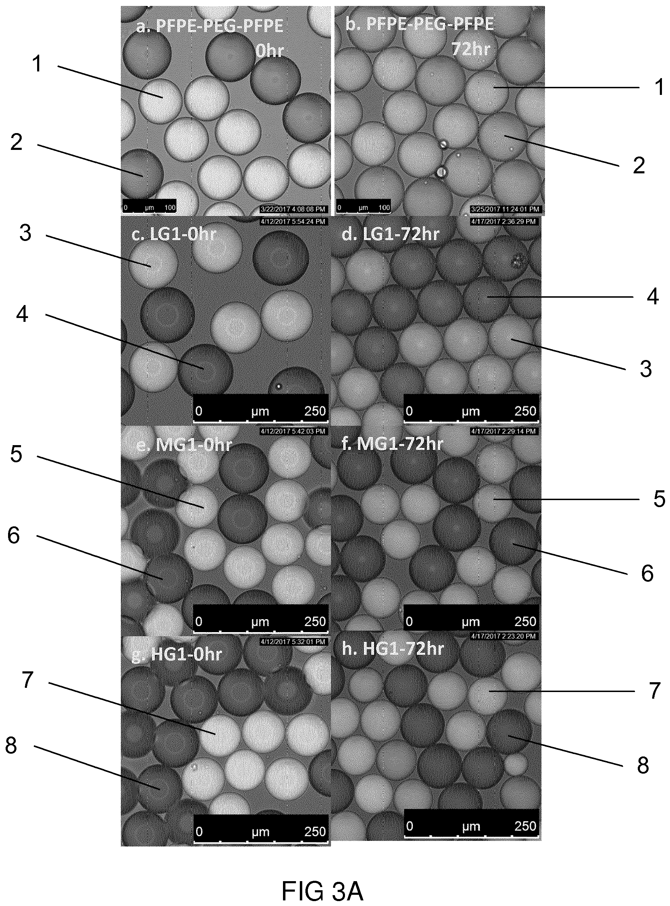

Panels a and b of Figure 3A show droplets stabilized by PFPE-PEG-PFPE. At 0 hours, the positive droplets 1 show a strong green fluorescence (light grey in Figure 3A), whereas the negative droplets 2 do not show any green fluorescence at all (dark grey in Figure 3A). After an incubation period of 72 hours, the fluorescence of the previously positive droplets 1 diminished, whereas the fluorescence of the previously negative droplets 2 increased. Rather, an almost equal distribution of the fluorescence between previously positive droplets 1 and previously negative droplets 2 could be observed.

Panels c and d of Figure 3A show droplets stabilized by LG1. At 0 hours, the positive droplets 3 show a strong green fluorescence (light grey in Figure 3A), whereas the negative droplets 4 do not show any green fluorescence at all (dark grey in Figure 3A). After an incubation period of 72 hours, the fluorescence of the positive droplets 3 almost remained unchanged, whereas the negative droplets 4 did still not show a significant fluorescence. This clearly indicates that LG1-stabilized droplets decrease the extent of cross-talking between individual droplets. Thus, if a substance is incorporated into an LG1-stabilized droplet, it remains within this droplet and is not transported to another droplet to a significant extent.

Panels e and f of Figure 3A show droplets stabilized by MG1. MG1 has a similar structure like LG1 but a higher molecular weight than LG1. It corresponds to formula (XII): 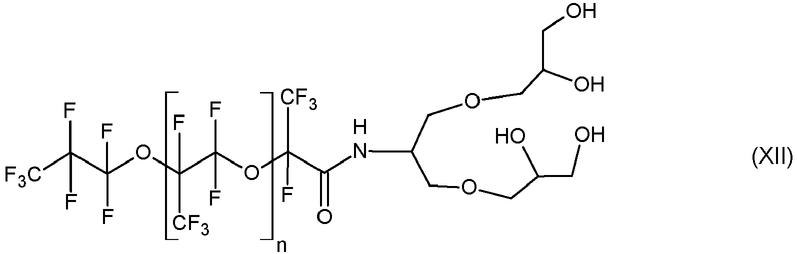 with n = 22 to 28

At 0 hours, the positive droplets 5 show a strong green fluorescence (light grey in Figure 3A), whereas the negative droplets 6 do not show any green fluorescence at all (dark grey in Figure 3A). After an incubation period of 72 hours, the fluorescence of the positive droplets 5 almost remained as strong as at 0 hours, whereas the negative droplets 6 did still not show any fluorescence. This clearly indicates that also MG1-stabilized droplets do not show cross-talking between individual droplets. Thus, if a substance is incorporated into an MG1-stabilized droplet, it remains within this droplet and is not transported to another droplet.

Panels g and h of Figure 3A show droplets stabilized by HG1. HG1 has a similar structure like LG1 and MG1 but an even higher molecular weight than MG1. It corresponds to formula (XIII): 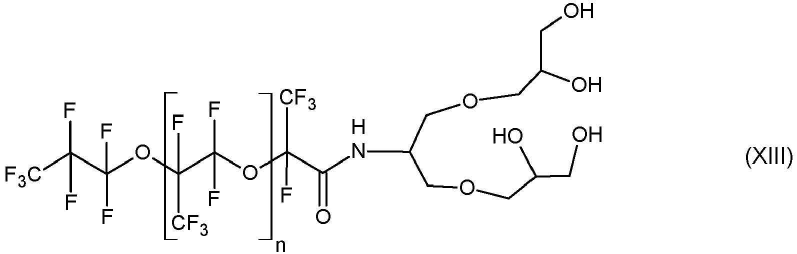 with n = 34 to 46

At 0 hours, the positive droplets 7 show a strong green fluorescence (light grey in Figure 3A), whereas the negative droplets 8 do not show any green fluorescence at all (dark grey in Figure 3A). After an incubation period of 72 hours, the fluorescence of the positive droplets 7 almost remained as strong as at 0 hours, whereas the negative droplets 8 did still not show any fluorescence. This clearly indicates that also HG1-stabilized droplets do not show cross-talking between individual droplets. Thus, if a substance is incorporated into an HG1-stabilized droplet, it remains within this droplet and is not transported to another droplet.

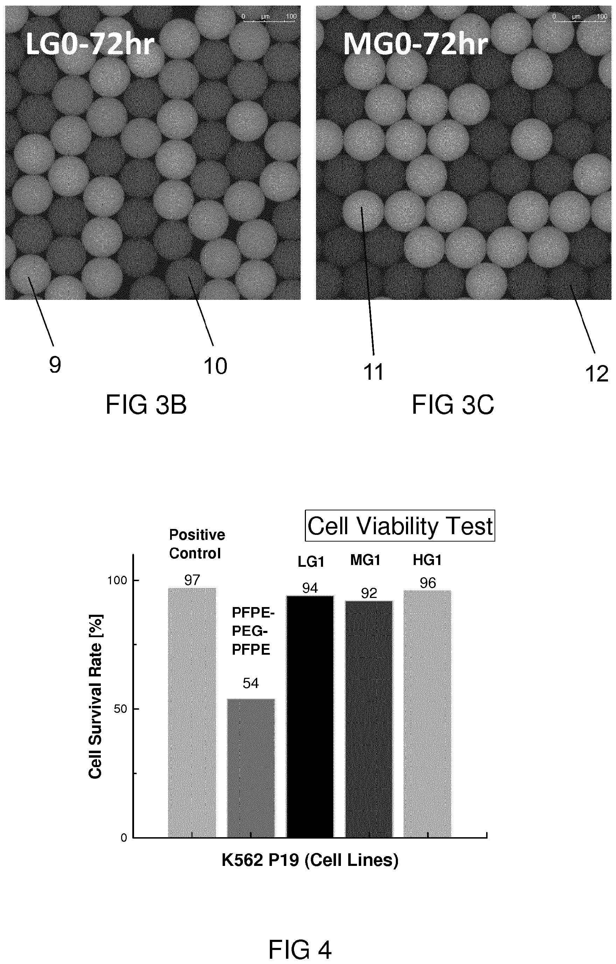

Figure 3B shows droplets stabilized by LG0. LG0 has a similar molecular structure like LG1, but only a single glycerol unit in the glycerol block. It corresponds to formula (XIV): 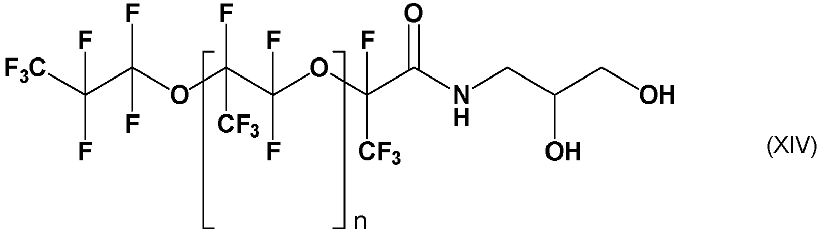 with n = 10 to 16

After an incubation period of 72 hours, positive droplets 9 showed a strong green fluorescence (light grey in Figure 3B), whereas the negative droplets 10 did not show any significant green fluorescence (dark grey in Figure 3B), Thereby, the fluorescence of the positive droplets 9 was almost as strong as directly after generating the droplets. This clearly indicates that also LG0-stabilized droplets did not show cross-talking between individual droplets. Thus, if a substance is incorporated into an LG0-stabilized droplet, it remains within this droplet and is not transported to another droplet.

Figure 3C shows droplets stabilized by MG0. MG0 has a similar structure like LG0 but a higher molecular weight than LG0. It corresponds to formula (XV): 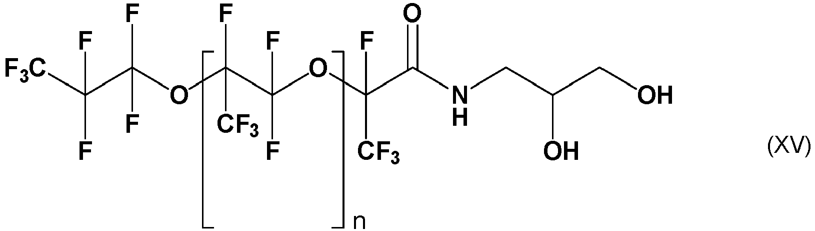 with n = 22 to 28

After an incubation period of 72 hours, positive droplets 11 showed a strong green fluorescence (light grey in Figure 3C), whereas the negative droplets 12 did not show any significant green fluorescence (dark grey in Figure 3C), Thereby, the fluorescence of the positive droplets 11 was almost as strong as directly after generating the droplets. This clearly indicates that also MG0-stabilized droplets did not show cross-talking between individual droplets. Thus, if a substance is incorporated into an MG0-stabilized droplet, it remains within this droplet and is not transported to another droplet.

### Example 3: Superior biocompatibility

The biocompatibility of LG1, MG1, and HG1 was assessed by means of a cell viability test. In the test, individual cells of the leukemia cell line K562 were encapsulated in the droplets (100µm). Fluorinated oil was used as the continuous phase and cell culture medium (DMEM) supplemented with 10% FBS was used as the aqueous phase. After 3 days of incubation, the droplets were destabilized by perfluorooctanol (PFO) and the cell viability was evaluated using a "live/dead staining kit". As shown in Figure 4, the cell viability in droplets stabilized by PFPE-PEG-PFPE (comparative example; negative control) was found to be 54 %, whereas the cell viability in LG1-, MG1-, and HG1-stabilized droplets was above 90 % for all three types of droplets stabilized by a diblock copolymer as described herein. The cell viability in LG1-, MG1-, and HG1-stabilized droplets almost achieved a viability rate of 97 % observed for cells incubated for 3 days in cell culture medium (DMEM) supplemented with 10% FBS (comparative example; positive control). No droplets were formed in this positive control.

### Example 4: Synthesis of an embodiment of a diblock copolymer

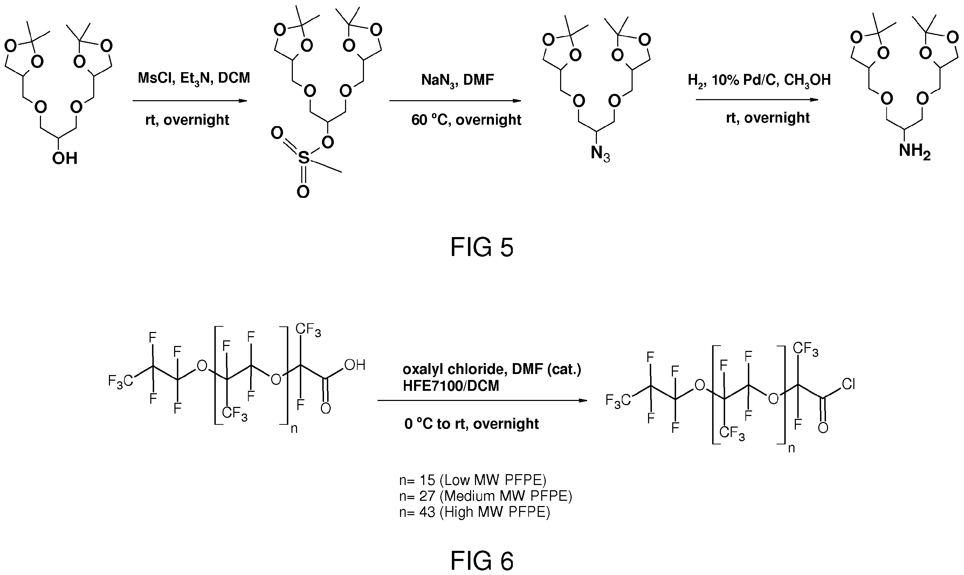

First, a protected oligoglycerol amine was manufactured according to the reaction scheme depicted in Figure 5. The starting material was an acetal protected [G1] polyglycerol dendron hydroxy that was dried in high vacuum at 60 °C overnight. Thereafter, G1 dendron-N₃ was prepared in a two-step process. First, the mesylation of the hydroxyl groups was carried out using 1.5 equivalent of methane sulfonyl chloride (MsCI) and two equivalents of triethyl amine in dichloromethane (DCM) at 0 °C to room temperature (RT). The mesylated compound was extracted in DCM and then dried under high vacuum (HV). Afterwards, the dried compound was dissolved in dimethylformamide (DMF) under argon, and NaN₃ (3 eq.) was added to the above reaction mixture. Substitution of the mesyl group with azide was carried out overnight at 70 °C. The final compound was obtained in 95% yield after extraction in DCM followed by drying in HV.

Finally, G1 dendron-N₃ was dissolved in methanol and hydrogenation of azide (-N₃) functional groups was conducted in presence of palladium catalyst (10% Pd/Charcoal) in a reactor with vigorous stirring for two days. The reduced compound was filtered off using a bed of cellite^{®}545. Subsequently, the solvent was removed and the residue was purified by column chromatography on silica gel using a hexane:isopropanol mixture as eluent. The thus purified product was obtained with 75% yield after drying in HV.

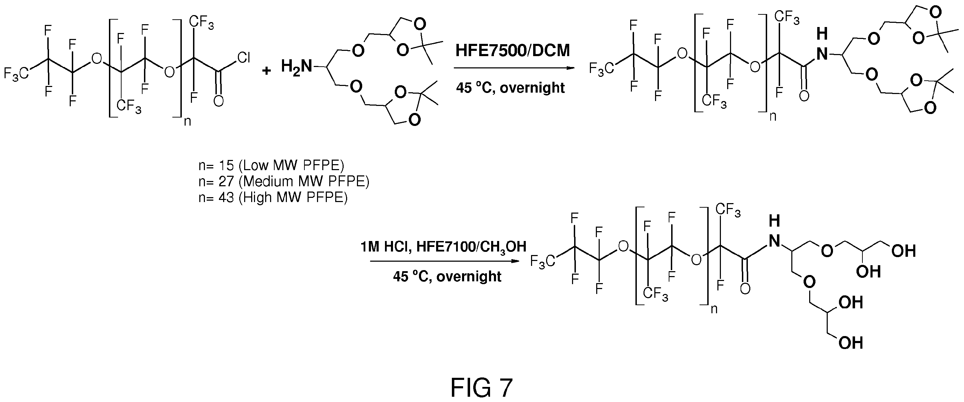

Separate from this, a perfluorinated polyether (PFPE) was activated to acid chloride overnight by reacting it with oxalyl chloride according to the reaction scheme depicted in Figure 6. The protected oligoglycerol amine synthesized according to the reaction scheme depicted in Figure 5 and the PFPE-COCI synthesized according to the reaction scheme depicted in Figure 6 were then reacted according to the reaction scheme in Figure 7 so that per-fluorinated diblock-copolymer based surfactants were synthesized in a three-step process. An amide bond is formed in the final step, wherein the amine group acts as linker between the PFPE block and the oligoglycerol block.

Firstly, a catalytic amount of DMF and oxalyl chloride (10 eq.) was added under argon atmosphere to a solution of perfluorinated polyethers (PFPE) in HFE-7100 to convert the terminal carboxylic group of PFPE to acid chloride. In the meantime, the argon filled balloon was removed and the outlet of a Schleck flask was connected to a Schleck line to let the gases escape during the reaction. After several minutes, the outlet valve was closed and an argon balloon was introduced again. The reaction mixture was stirred overnight at RT. Next, HFE-7100, DMF, and all volatile gases were removed under HV using an addition cold trap. The PFPE species Krytox 157 FSH, Krytox 157 FSM, and Krytox 157 FSL were used as high, medium, and low molecular weight block, respectively, and all of them were activated to acid chloride in a similar fashion.

Next, the viscous PFPE acid chloride was diluted adding small amount of HFE-7100 oil. Then a freshly prepared solution of G1 dendron-NH₂ (dried under HV at 50 °C) (1.07 eq. to PFPE acid chloride) in dry DCM was mixed with HFE-7100 oil in a ratio of 1:0.75. Later, dropwise addition of G1 dendron-NH₂ solution into the activated Krytox compound was performed under inert condition using a dropping funnel over a period of ~1 h. The reaction mixture was then refluxed overnight at 45-50 °C. The crude product was transferred into a one neck round bottom flask. DCM was evaporated under reduced pressure followed by removal of HFE-7100 in HV using an additional cold trap. Lastly, the dried viscous compound was dissolved in a mixture of HFE-7100 and methanol solution and deprotection acetal groups was carried out overnight at 50 °C adding 0.5-1 mL of 1M HCI solution to the above reaction mixture. The thus prepared high, medium, and low MW diblock-copolymer surfactants (having 15, 27 or 43 repeating units) were obtained with a yield of 75-85% after drying in HV.

### Example 5: Characterization of the dendronized fluorosurfactants

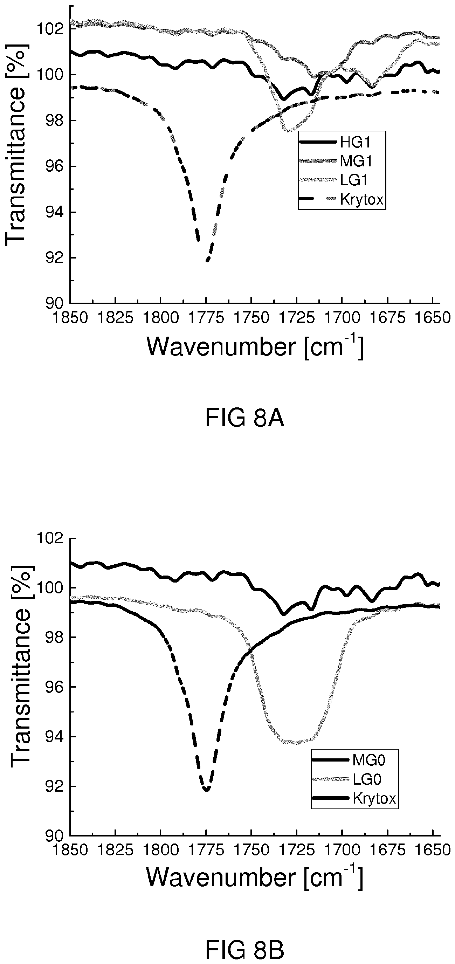

The successfully synthesized surfactants with different generation oligoglycerols, e.g. monoglycerol (G0) and triglycerol (G1), and Krytox blocks were characterized by Fourier transform infrared (FT-IR) spectroscopy. Figure 8A shows the FT-IR spectra of HG1, MG1, and LG1. Figure 8B shows the FT-IR spectra of MG0 and LG0.

Typically, the terminal carbonyl stretch (C=O) of the starting perfluorinated block (PFPE) shows, irrespective of its molecular weight, an absorbance peak at 1775 cm⁻¹. Therefore, a representative spectrum of the PFPE polymer is presented by a dashed line. On the other hand, the absorption peaks at about 1675 cm⁻¹ and 1725 cm⁻¹ indicate the amide stretch vibration (-CONH-) in the synthesized surfactants when G0 and G1 dendrons are incorporated into the PFPE block.

### Example 6: Synthesis of a protected dithiolated G1-OH dendron

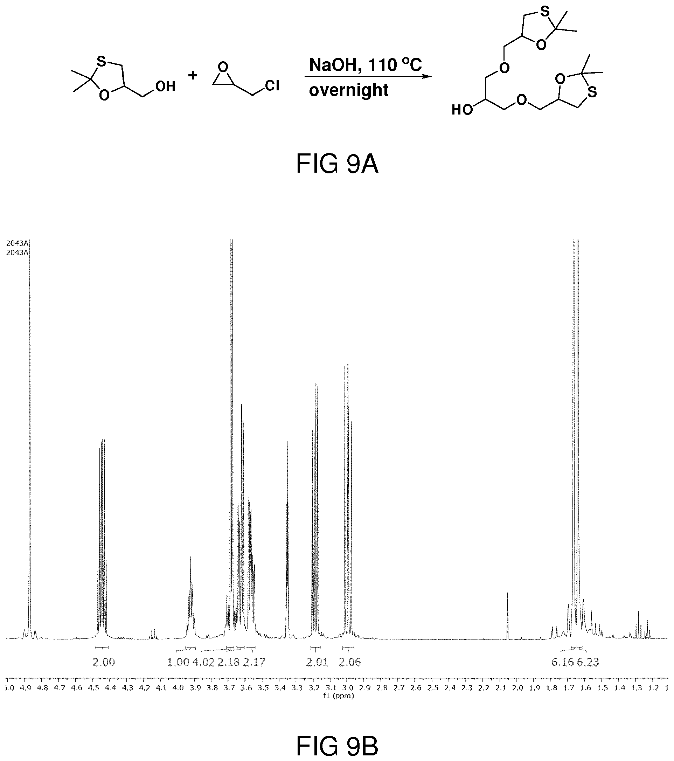

A protected dithiolated G1 dendron [1,3-bis((2,2-dimethyl-1,3-oxathiolan-5-yl)methoxy)propan-2-ol] was synthesized using epichlorohydrin and (2,2-dimethyl-1,3-oxathiolan-5-yl)methanol according to the reaction scheme depicted in Figure 9A. This synthetic procedure includes a reaction of one mole equivalent of epichlorohydrin with four mole equivalents of (2,2-dimethyl-1,3-oxathiolan-5-yl)methanol and a base e.g. sodium hydroxide (NaOH). The 1H-NMR spectrum depicted in Figure 9B confirms the successful synthesis of the acetal-protected dithiolated G1-OH dendron. The secondary hydroxyl (-OH) group can itself be a functional group and be modified to any other functional groups, e.g. amine, azide, halogen etc. Moreover, after acetal deprotection - apart from the possibility to modify the hydroxyl groups to other functional groups - the dithiols can be protected with any suitable thiol protecting groups, e.g. with 2,2'-dipyridyldisulfide.

Finally, both monothiolated G0 dendron ((2,2-dimethyl-1,3-oxathiolan-5-yl)methanol) and dithiolated G1 dendron can be used to prepare a variety of dendronized diblock copolymer fluorosurfactants. The novel protected dithiolated G1-OH dendron can also be utilized to formulate other nanostructures. In particular, micelles, vesicles, liposomes, and oil-in-water (O/W) emulsions can be also obtained using monothiolated and dithiolated G1 dendron as polar head groups along with any fluorinated block e.g. fluorinated alkyl chain, PFPE, and classical alkyl chain derivatives.

### Example 7: Temperature Stability (PCR reaction in droplets)

This example was performed using the same reaction conditions as in Example 1.

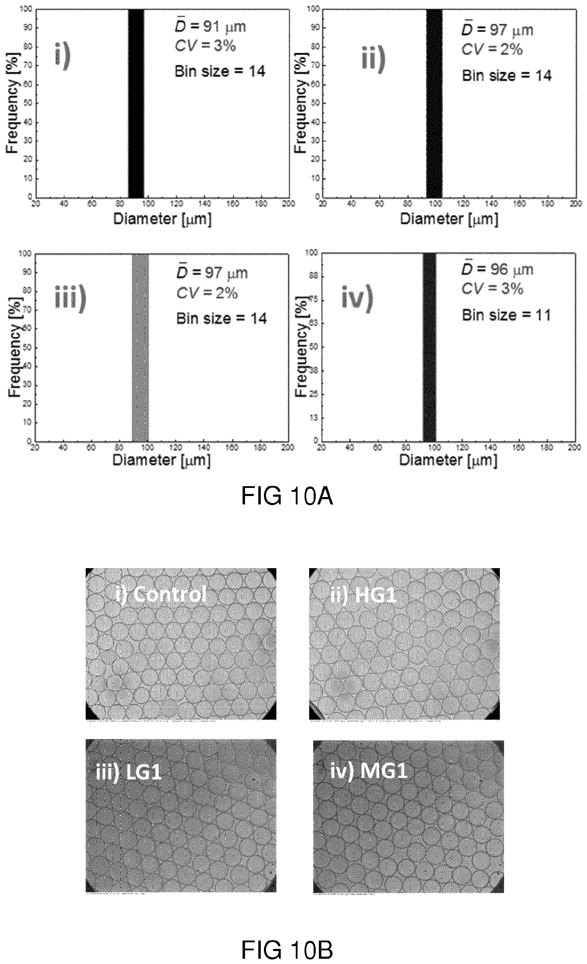

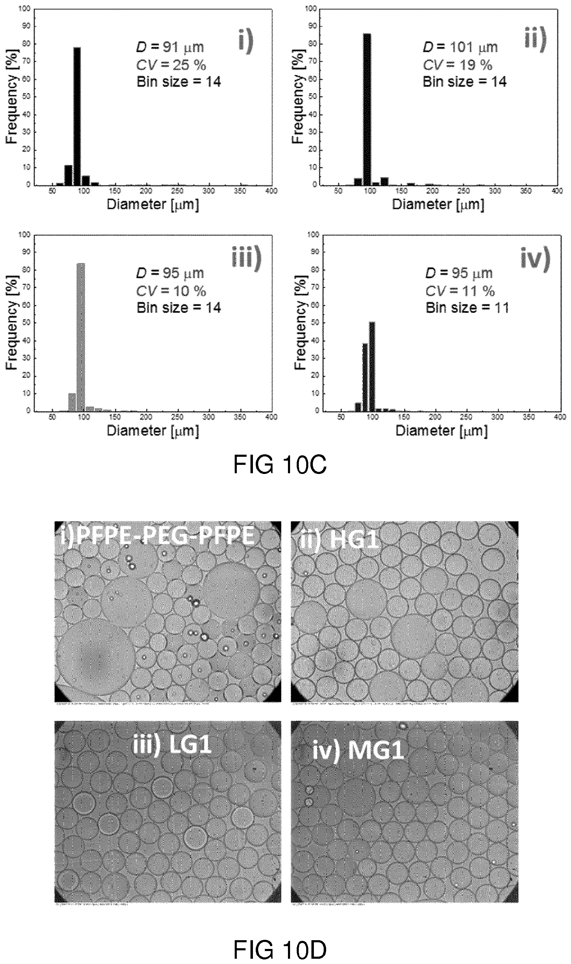

Different surfactants based on G1 dendrons along with PFPE-PEG-PFPE as a control were used to perform the PCR reaction. Droplets having a diameter of approximately 100 µm were prepared with a coefficient of variation (CV) of around 2 % in all cases. The corresponding size distributions before performing a PCR are depicted in Figure 10A. Figure 10B shows microscopic pictures of these droplets.

After PCR test, HG1, MG1, and LG1 surfactants demonstrated excellent single drop resolution stability with CVs of about 19%, 10 %, and 11 %, respectively. The corresponding size distributions after performing the PCR test are depicted in Figure 10C. On the other hand, the control group showed instability due to the PCR reaction at elevated temperature. To be more specific, it exhibited a 2.5 times higher CV (25 %) as compared to MG1 and LG1. Figure 10D shows microscopic pictures of the droplets after the PCR test.

### Example 8: Synthesis of microgel template

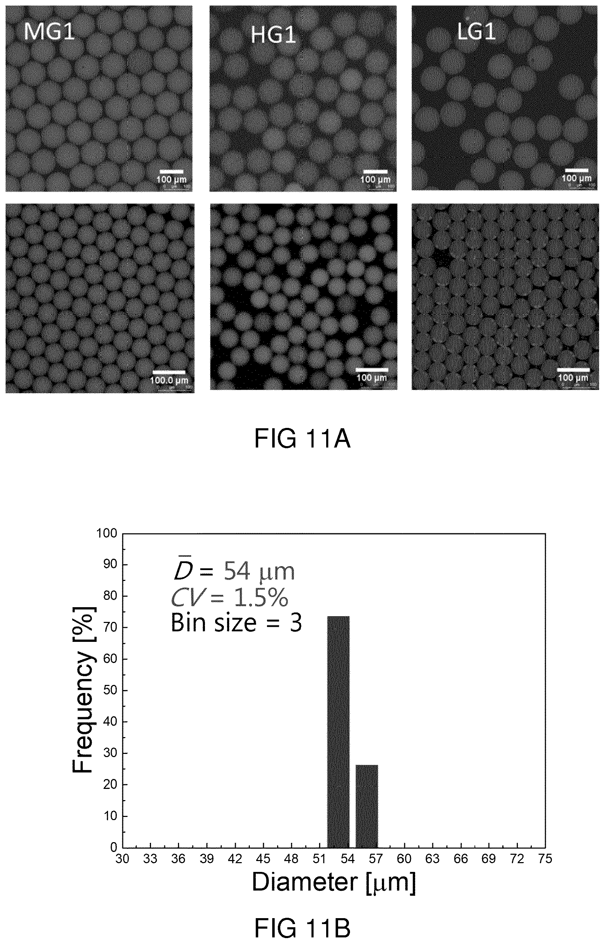

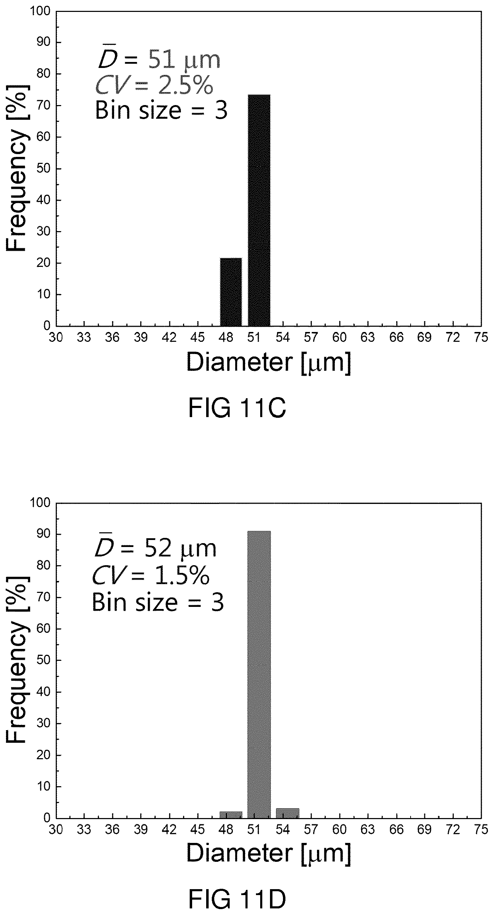

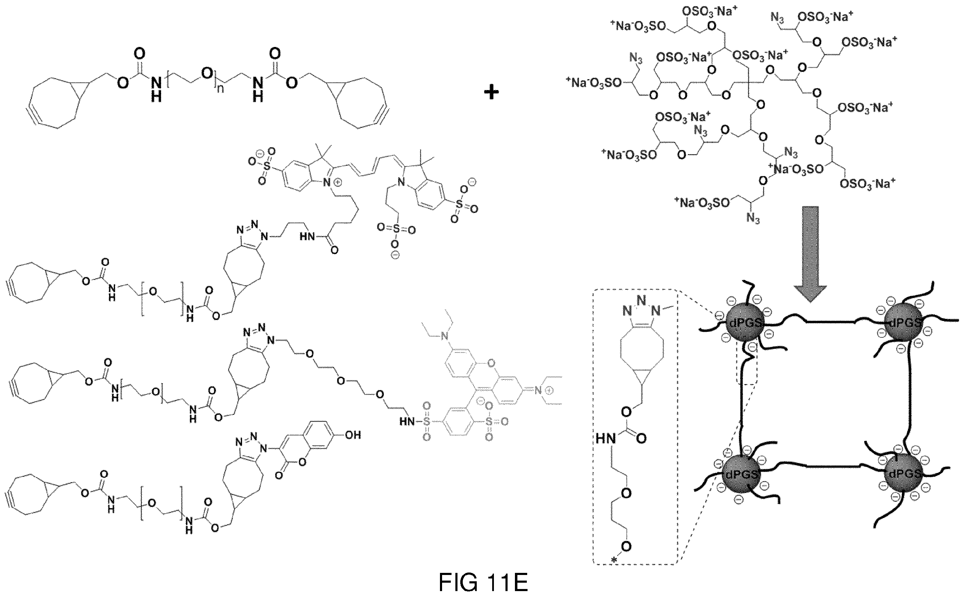

Triglycerol polar head group based surfactants were used to prepare microgel particles. A strain-promoted azide alkyne cycloaddition (SPAAC) reaction was performed to form a three-dimensional cross-linked network, i.e. the microgels. Different dye labelled cross linkers were used to identify different microparticles prepared incorporating HG1, MG1, and LG1 surfactants. However, the formation of microgel particles is not limited to the macromolecules used in this work. Ideally, any polymeric material suitable for microparticle formation in an aqueous environment can be used to make templates of different sizes ranging from one micrometer to several hundred micrometers using the diblock copolymer surfactants as the stabilizer of the aqueous phase. Here, microparticles with a diameter of about 50 µm were prepared with a CV of about 2% with all three types of fluorosurfactants (HG1, MG1, and LG1). Figure 11A shows templated microgels before surfactant removal (lower panels) and after surfactant removal (upper panels). Figures 11B to 11D show the corresponding size distributions, namely of MG1 (Figure 11B), HG1 (Figure 11C), and of LG 1 (Figure 11D). Figure 11E shows precursor macromolecules that were used to prepare the microgel templates and a reaction scheme of the applied chemical reaction.

### Example 9: Drug screening mimetic biological assay (Cross talking)

To determine cross-talking phenomena among droplets under biological environment Human Embryonic Kidney cell 293 (HEK 293) was genetically modified using Piggybac transposition gene editing tool. Two plasmid vectors, namely hyPBase (vector was obtained from the Wellcome Trust Sanger Institute, UK) and XLone-GFP (vector was obtained from Xiaojun Lian Lab (Pennsylvania State University, USA) through Addgene) were incorporated to insert a gene of interest (GOI) in the host cell's genome. The genetically modified stable HEK293 cells can express a fluorescent protein, e.g. green fluorescence protein (GFP), only in presence of a suitable drug, e.g. doxycycline (Dox). Herein, as positive control, HEK293 cells were dispersed in doxycycline-containing cell culture medium. Afterwards, drops having a diameter of about 80 µm were prepared.

To investigate the cross-talking phenomena, two different sets of drops were co-mixed, wherein one set of drops contained the Dox-inducible HEK293 cells and the other set of drops contained doxycycline solution. On day 1, images of drops from positive control and mixed drop populations were taken using fluorescence microscope.

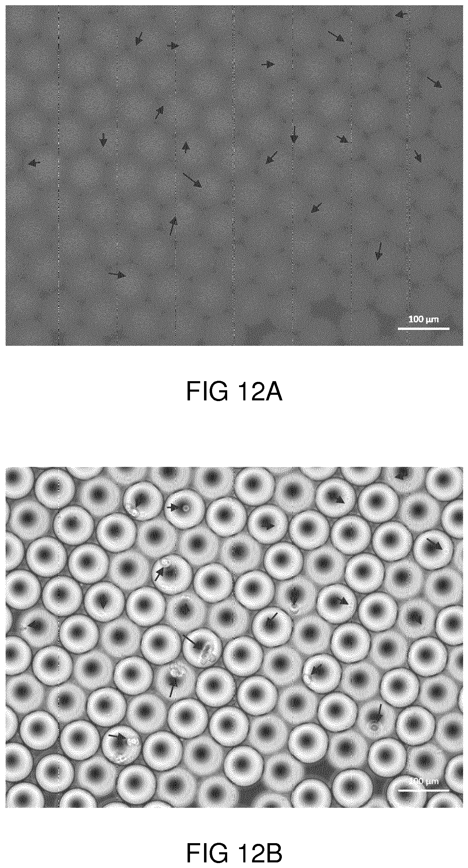

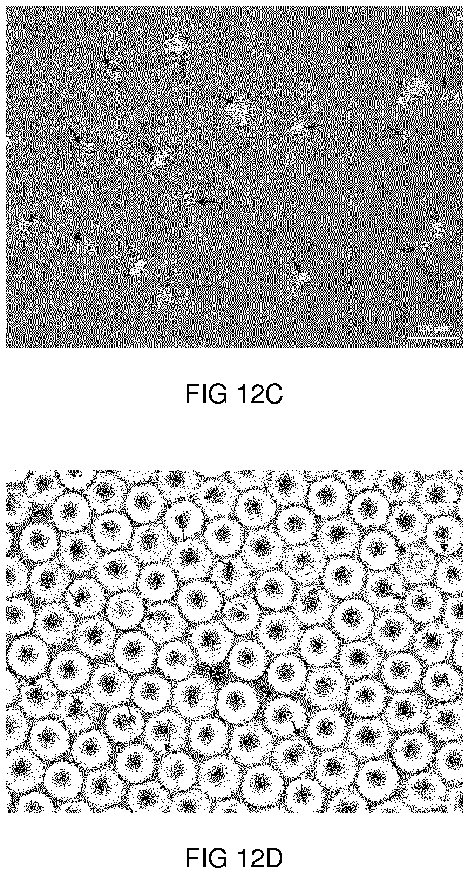

Figure 12A shows a fluorescence image and Figure 12B an overlay of bright field and fluorescence of mixed drop populations. Figure 12C shows a fluorescence image and Figure 12D an overlay of bright field and fluorescence of the positive control. The arrows in the images are used only to guide the eyes to facilitate distinguishing GFP+ from GFP- cells.

## Claims

1. Diblock copolymer consisting of a first block, a second block, and a linker, wherein the second block is covalently bound to the first block by the linker, wherein
• the first block is a glycerol block comprising 1 to 10 glycerol subunits that are optionally substituted, and
• the second block is a superhydrophobic block comprising a perfluoroether residue having at least 20 carbon atoms.

2. Diblock copolymer according to claim 1, wherein the glycerol block comprises an optionally substituted oligoglycerol having 3 to 10 glycerol subunits.

3. Diblock copolymer according to claim 2, wherein the oligoglycerol is a branched oligoglycerol.

4. Diblock copolymer according to any of the preceding claims, wherein the glycerol block corresponds to any of general formulae (I) to (VIII) 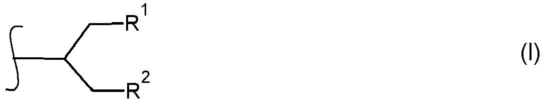 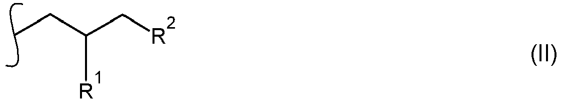 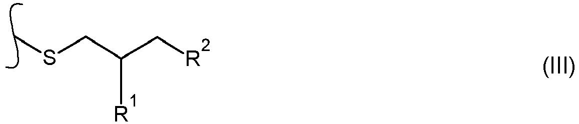 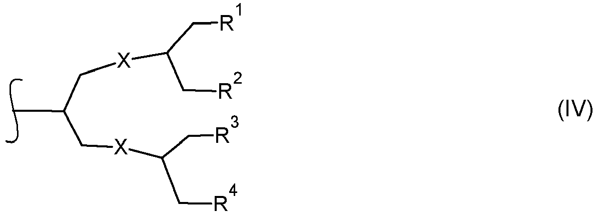 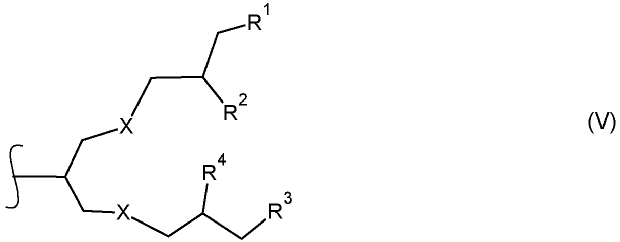 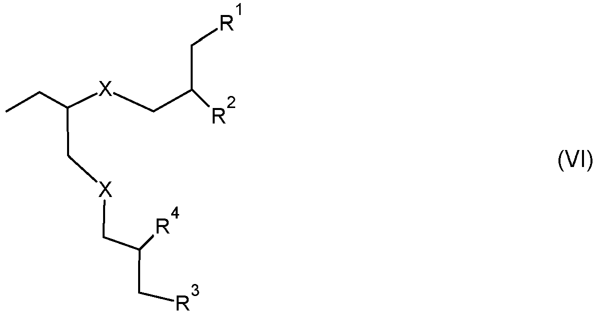 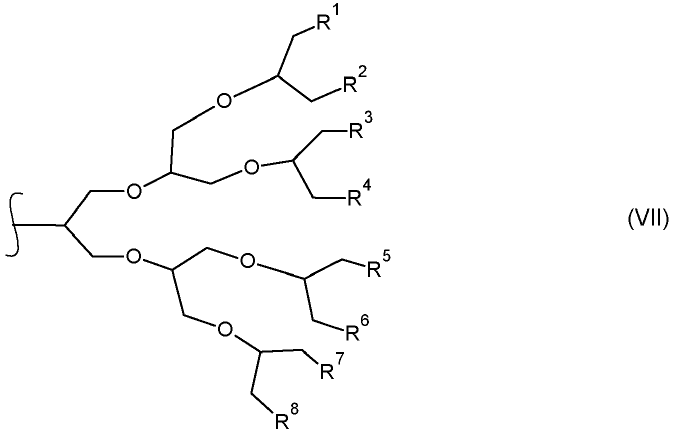 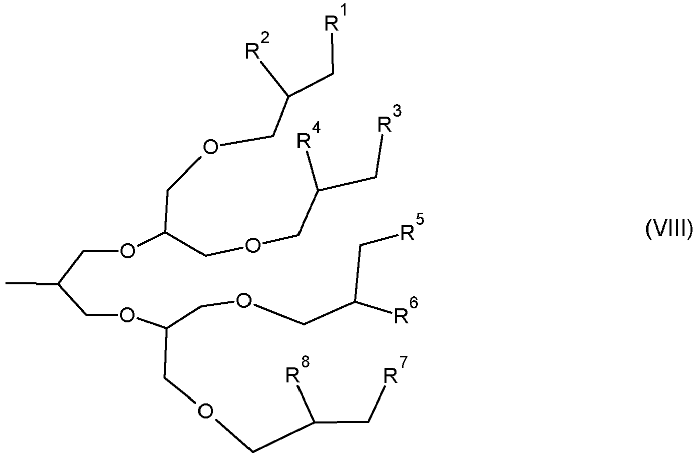
wherein R¹, R², R³, R⁴, R⁵, R⁶, R⁷, R⁸ is independently from each other -SH, -CH₃, -CH₂CH₃, -NH₂, -OH, -OCH₃, -OCH₂CH₃, -OCH₂OCH₂CH₃, -NH₃⁺, -OPO₃⁻(CH₂)₂N⁺(CH₃)₃, -(CH₃)₂N⁺(CH₂)₂OPO₂⁻(OCH₃), -(CH₃)₂N⁺(CH₂)₂OPO₂⁻(OH), or -OSO₃⁻,
and X is O or S.

5. Diblock copolymer according to any of the preceding claims, wherein the superhydrophobic block comprises two carbon-containing chains that are bound to the same linker but are not covalently crosslinked to each other.

6. Diblock copolymer according to any of the preceding claims, wherein the superhydrophobic block comprises a residue according to general formula (IX), 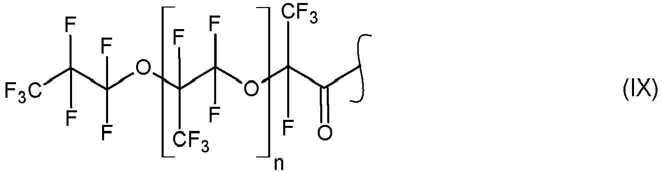 wherein n = 10 to 50.

7. Diblock copolymer according to any of the preceding claims, wherein the linker is chosen from the group consisting of amines, amides, oxygen, and an alkyl residue having 1 to 20 carbon atoms, wherein a hydrocarbon chain of the alkyl residue can be interrupted by one or more oxygen, nitrogen and/or sulfur atoms and/or can be substituted by a group comprising one or more oxygen, nitrogen and/or sulfur atoms.

8. Diblock copolymer according to any of the preceding claims, wherein the linker is not a methylene group.

9. Method for manufacturing a diblock copolymer according to any of the preceding claims, comprising reacting an oligoglycerol amine comprising protected hydroxyl groups with a perfluorinated polyether acid chloride in an organic solvent, and deprotecting the hydroxyl groups of the obtained diblock copolymer.

10. Method according to claim 9, wherein the oligoglycerol amine comprising protected hydroxyl groups is manufactured by mesylating an oligoglycerol comprising protected hydroxyl groups and at least one unprotected hydroxyl group to introduce a mesyl group into the oligoglycerol, substituting the mesyl group with an azide group and hydrogenating the azide group to obtain the oligoglycerol amine comprising protected hydroxyl groups.

11. Method according to claim 9 or 10, wherein the perfluorinated polyether acid chloride is manufactured by reacting a perfluorinated polyether with oxalyl chloride.

12. Droplet comprising a plurality of diblock copolymers according to any of claims 1 to 7.

13. Droplet according to claim 12, wherein it comprises a mixture of structurally different diblock copolymers.

14. Use of a droplet according to claim 12 or 13 for performing a polymerase chain reaction in an interior of the droplet.

15. Use of a droplet according to claim 12 or 13 for in vitro testing an effect of a substance on a cell incorporated in the droplet.

## Patentansprüche

1. Diblock-Copolymer, bestehend aus einem ersten Block, einem zweiten Block und einem Linker, wobei der zweite Block über den Linker kovalent an den ersten Block gebunden ist, wobei
• der erste Block ein Glycerinblock ist, der 1 bis 10 Glycerinuntereinheiten umfasst, die optional substituiert sind, und
• der zweite Block ein superhydrophober Block ist, der einen Perfluorether-Rest mit zumindest 20 Kohlenstoffatomen umfasst.

2. Diblock-Copolymer nach Anspruch 1, wobei der Glycerinblock ein optional substituiertes Oligoglycerin mit 3 bis 10 Glycerinuntereinheiten umfasst.

3. Diblock-Copolymer nach Anspruch 2, wobei das Oligoglycerin ein verzweigtes Oligoglycerin ist.

4. Diblock-Copolymer nach einem der vorstehenden Ansprüche, wobei der Glycerinblock einer der allgemeinen Formeln (I) bis (VIII) entspricht: 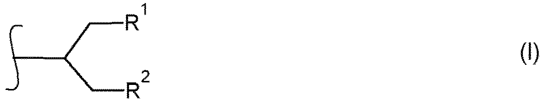 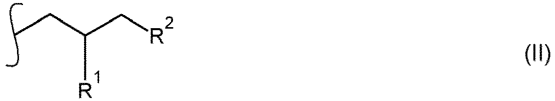 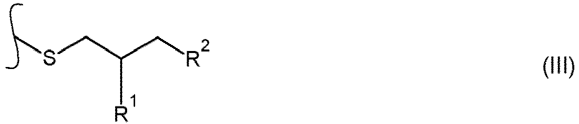 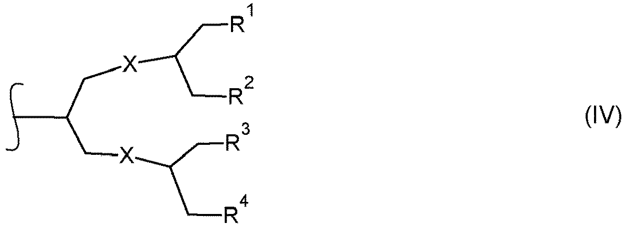 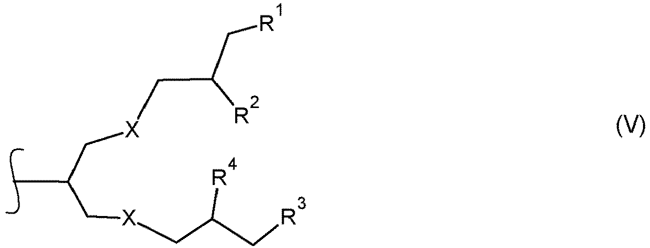 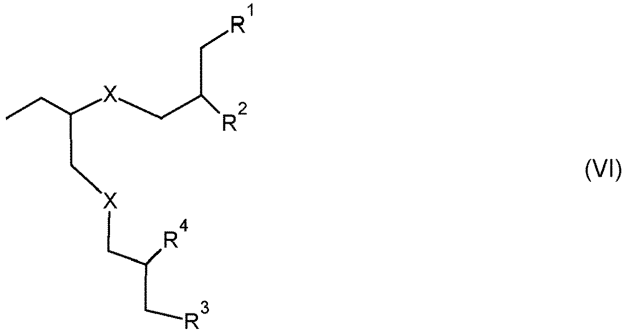 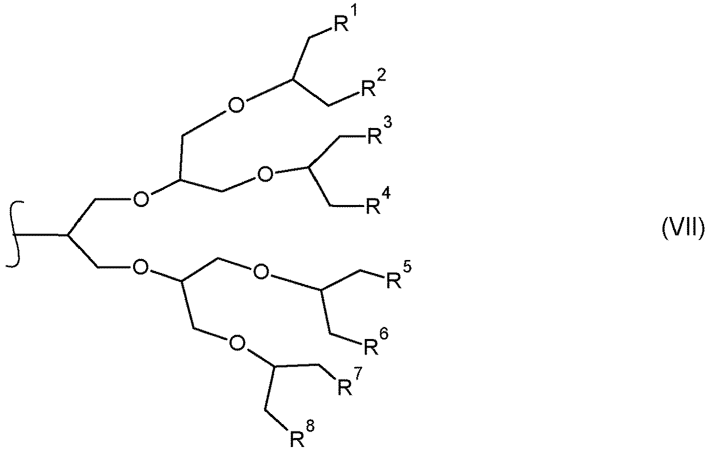 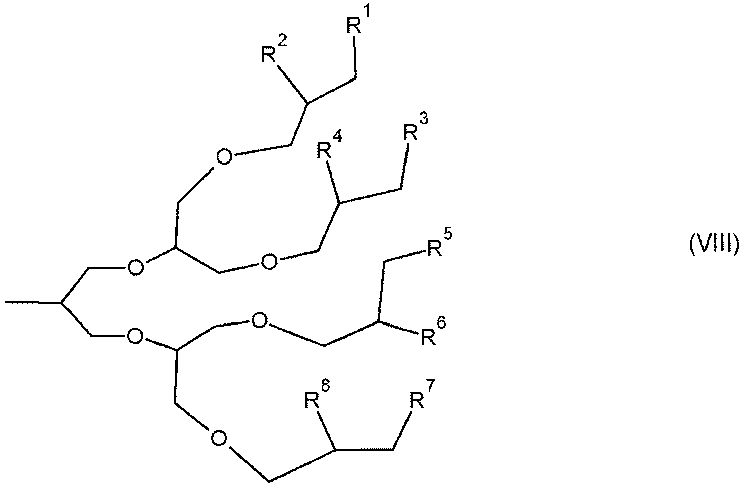 wobei R¹, R², R³, R⁴, R⁵, R⁶, R⁷, R⁸ unabhängig voneinander -SH, -CH₃, -CH₂CH₃, -NH₂, -OH, -OCH₃, -OCH₂CH₃, -OCH₂OCH₂CH₃, -NH₃⁺, -OPO₃⁻, (CH₂)₂N⁺(CH₃)₃, -(CH₃)₂N⁺(CH₂)₂OPO₂⁻(OCH₃), -(CH₃)₂N⁺(CH₂)₂OPO₂⁻(OH) oder -OSO₃⁻ ist und X O oder S ist.

5. Diblock-Copolymer nach einem der vorstehenden Ansprüche, wobei der superhydrophobe Block zwei kohlenstoffhaltige Ketten umfasst, die an denselben Linker gebunden sind, jedoch nicht kovalent miteinander vernetzt sind.

6. Diblock-Copolymer nach einem der vorstehenden Ansprüche, wobei der superhydrophobe Block einen Rest gemäß der allgemeinen Formel (IX) aufweist: 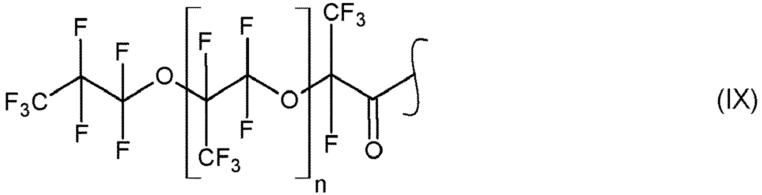 wobei n = 10 bis 50.

7. Diblock-Copolymer nach einem der vorstehenden Ansprüche,
wobei der Linker ausgewählt ist aus der Gruppe bestehend aus Aminen, Amiden, Sauerstoff und einem Alkylrest mit 1 bis 20 Kohlenstoffatomen, wobei eine Kohlenwasserstoffkette des Alkylrestes durch ein oder mehrere Sauerstoff-, Stickstoff- und/oder Schwefelatome unterbrochen sein kann und/oder durch eine Gruppe substituiert sein kann, die ein oder mehrere Sauerstoff-, Stickstoff- und/oder Schwefelatome umfasst.

8. Diblock-Copolymer nach einem der vorstehenden Ansprüche, wobei der Linker keine Methylengruppe ist.

9. Verfahren zur Herstellung eines Diblock-Copolymers nach einem der vorstehenden Ansprüche, umfassend das Umsetzen eines Oligoglycerin-Amins, das geschützte Hydroxylgruppen aufweist, mit einem perfluorierten Polyethersäurechlorid in einem organischen Lösungsmittel, und das Entschützen der Hydroxylgruppen des erhaltenen Diblock-Copolymers.

10. Verfahren nach Anspruch 9, wobei das Oligoglycerinamin, das geschützte Hydroxylgruppen aufweist, durch Mesylierung eines Oligoglycerins, das geschützte Hydroxylgruppen und zumindest eine ungeschützte Hydroxylgruppe zur Einführung einer Mesylgruppe in das Oligoglycerin umfasst, durch Substitution der Mesylgruppe durch eine Azidgruppe und durch Hydrierung der Azidgruppe hergestellt wird, um das Oligoglycerinamin, das geschützte Hydroxylgruppen aufweist, zu erhalten.

11. Verfahren nach Anspruch 9 oder 10, wobei das perfluorierte Polyethersäurechlorid durch Umsetzen eines perfluorierten Polyethers mit Oxalylchlorid hergestellt wird.

12. Tröpfchen, umfassend eine Vielzahl von Diblock-Copolymeren nach einem der Ansprüche 1 bis 7.

13. Tröpfchen nach Anspruch 12, wobei es ein Gemisch aus strukturell unterschiedlichen Diblock-Copolymeren umfasst.

14. Verwendung eines Tröpfchens nach Anspruch 12 oder 13 zur Durchführung einer Polymerase-Kettenreaktion in einem Innern des Tröpfchens.

15. Verwendung eines Tröpfchens nach Anspruch 12 oder 13 zur In-vitro-Prüfung einer Wirkung einer Substanz auf eine in dem Tröpfchen enthaltenen Zelle.

## Revendications

1. Copolymère dibloc constitué d'un premier bloc, un second bloc et un lieur, dans lequel le second bloc est lié de manière covalente au premier bloc par le lieur, dans lequel
• le premier bloc est un bloc glycérol comprenant 1 à 10 sous-unités glycérol qui sont éventuellement substituées, et
• le second bloc est un bloc superhydrophobe comprenant un résidu perfluoroéther présentant au moins 20 atomes de carbone.

2. Copolymère dibloc selon la revendication 1, dans lequel le bloc glycérol comprend un oligoglycérol éventuellement substitué présentant 3 à 10 sous-unités glycérol.

3. Copolymère dibloc selon la revendication 2, dans lequel l'oligoglycérol est un oligoglycérol ramifié.

4. Copolymère dibloc selon l'une quelconque des revendications précédentes, dans lequel le bloc glycérol correspond à l'une quelconque des formules générales (I) à (VIII) 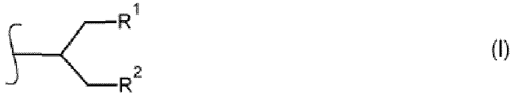 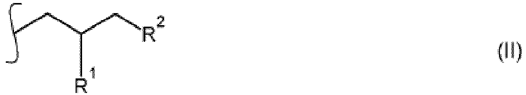 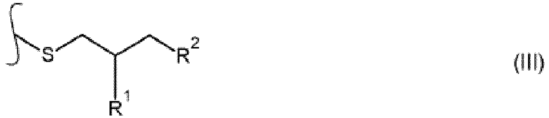 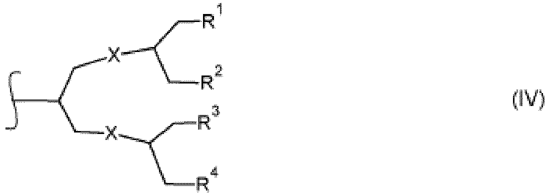 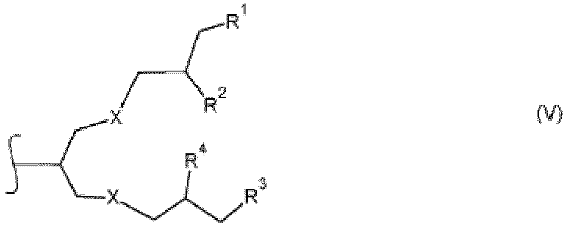 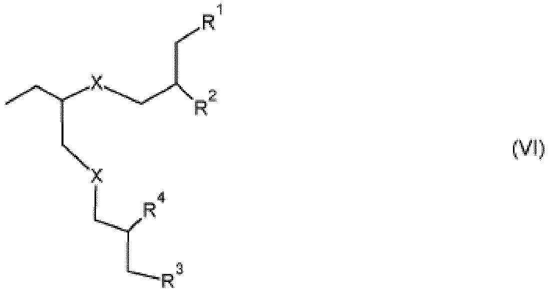 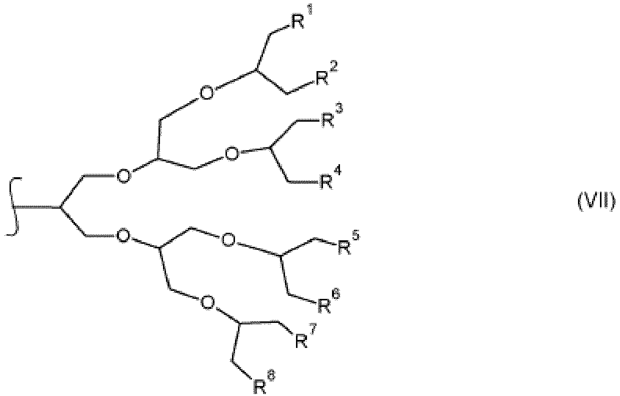 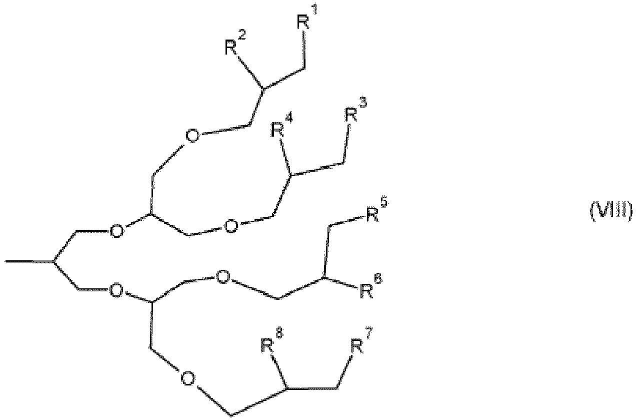
dans lequel R¹, R², R³, R⁴, R⁵, R⁶, R⁷, R⁸ est indépendamment l'un de l'autre - SH, -CH₃, -CH₂CH₃, -NH₂, -OH, -OCH₃, -OCH₂CH₃, -OCH₂OCH₂CH₃, -NH₃⁺, - OPO₃⁻(CH₂)₂N⁺(CH₃)₃, -(CH₃)₂N⁺(CH₂)₂OPO₂⁻(OCH₃), - (CH₃)₂N⁺(CH₂)₂OPO₂⁻(OH) ou -OSO₃⁻,
et X est O ou S.

5. Copolymère dibloc selon l'une quelconque des revendications précédentes, dans lequel le bloc superhydrophobe comprend deux chaînes contenant du carbone, qui sont liées au même lieur mais ne sont pas réticulées de manière covalente l'une à l'autre.

6. Copolymère dibloc selon l'une quelconque des revendications précédentes, dans lequel le bloc superhydrophobe comprend un résidu selon la formule générale (IX) 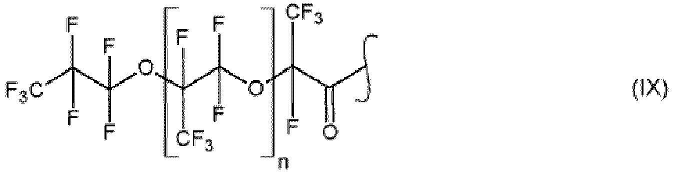 dans lequel n = 10 à 50.

7. Copolymère dibloc selon l'une quelconque des revendications précédentes, dans lequel le lieur est choisi parmi le groupe constitué par des amines, des amides, de l'oxygène et un résidu alkyle présentant 1 à 20 atomes de carbone, dans lequel une chaîne d'hydrocarbures du résidu alkyle peut être interrompue par un ou plusieurs atomes d'oxygène, d'azote et/ou de soufre et/ou peut être substituée par un groupe comprenant un ou plusieurs atomes d'oxygène, d'azote et/ou de soufre.

8. Copolymère dibloc selon l'une quelconque des revendications précédentes, dans lequel le lieur n'est pas un groupe méthylène.

9. Procédé de fabrication d'un copolymère dibloc selon l'une quelconque des revendications précédentes, comprenant la réaction d'une amine d'oligoglycérol comprenant des groupes hydroxyle protégés avec un chlorure d'acide de polyéther perfluoré dans un solvant organique, et la déprotection des groupes hydroxyle du copolymère dibloc obtenu.

10. Procédé selon la revendication 9, dans lequel l'amine d'oligoglycérol comprenant des groupes hydroxyle protégés est fabriquée par méthylation d'un oligoglycérol comprenant des groupes hydroxyle protégés et au moins un groupe hydroxyle non protégé pour introduire un groupe mésyle dans l'oligoglycérol, par substitution du groupe mésyle par un groupe azide et par hydrogénation du groupe azide pour obtenir l'amine d'oligoglycérol comprenant des groupes hydroxyle protégés.

11. Procédé selon la revendication 9 ou 10, dans lequel l'acide de chlorure de polyéther perfluoré est fabriqué par réaction d'un polyéther perfluoré avec du chlorure d'oxalyle.

12. Gouttelette comprenant une pluralité de copolymères diblocs selon l'une quelconque des revendications 1 à 7.

13. Gouttelette selon la revendication 12, dans laquelle elle comprend un mélange de copolymères diblocs structurellement différents.

14. Utilisation d'une gouttelette selon la revendication 12 ou 13 pour réaliser une réaction en chaîne par polymérase dans un intérieur de la gouttelette.

15. Utilisation d'une gouttelette selon la revendication 12 ou 13 pour tester in vitro un effet d'une substance sur une cellule incorporée dans la gouttelette.
